# EUROPÄISCHE PATENTSCHRIFT

(11) **EP 3 890 798 B1**
(45) Veröffentlichungstag und Bekanntmachung des Hinweises auf die Patenterteilung: **19.02.2025**
(21) Anmeldenummer: 19835598.4
(22) Anmeldetag: 04.12.2019
(51) Int. Cl.: A61M 1/00

(54) **MEDIZINTECHNISCHE SPÜLPUMPE MIT ZWEI ABSAUGLEITUNGEN**
MEDICAL-TECHNOLOGY IRRIGATION PUMP WITH TWO SUCTION LINES
POMPE D'IRRIGATION DE TECHNOLOGIE MÉDICALE AVEC DEUX CONDUITES D'ASPIRATION

(30) Priorität: 07.12.2018 DE 102018009537; 05.07.2019 DE 102019004629
(43) Veröffentlichungstag der Anmeldung: 13.10.2021
(73) Patentinhaber: W.O.M. World of Medicine GmbH, 10587 Berlin (DE)
(72) Erfinder: HUNGER, Christian, 16518 Oranienburg (DE); TARTIVEL, Lucille, 10245 Berlin (DE); CARSTENS, Jan Hendrik, 10405 Berlin (DE); GELBERT, Nils, 12555 Berlin (DE); SCHNÜTTGEN, Stan, 10179 Berlin (DE); RATH, Oliver, 10179 Berlin (DE); CHRISTMANN, Thomas, 13187 Berlin (DE); PLESSNER, Mike, 14662 Friesack (DE)
(74) Vertreter: Gulde & Partner
(86) Internationale Anmeldenummer: PCT/DE2019/000312
(87) Internationale Veröffentlichungsnummer: WO 2020/114534

(56) Entgegenhaltungen:
- EP-A2- 0 173 816
- EP-B1- 1 374 925
- US-A1- 2012 090 620

## Beschreibung

Die vorliegende Erfindung betrifft eine Vorrichtung zum Durchspülen einer Körperhöhle mit einem Fluid, wobei das Fluid mittels zweier Leitungen aus der Körperhöhle abgepumpt wird. Die jeweilige Pumpleistung wird über steuerbare Quetschventile geregelt, wobei die beiden Quetschventile miteinander antiparallel gekoppelt sind.

### Anwendungsgebiet der Erfindung

Es ist bekannt bei endoskopischen Untersuchungen und insbesondere bei therapeutischen Eingriffen, Flüssigkeitsspülungen vorzunehmen. Hierbei wird die Körperhöhle (z.B. ein Gelenkhohlraum, eine Blase oder eine Gebärmutter) mit einer Flüssigkeit (z.B. Kochsalzlösung) durchspült. Hierzu kann eine Spülflüssigkeit aus einem Vorratsbehälter über eine Pumpe, z.B. eine Rollenradpumpe, in die Körperhöhle gepumpt werden. Die Spülflüssigkeit kann im einfachsten Fall durch eine Öffnung aus dem Hohlraum wieder auslaufen. In der Regel werden jedoch Saugpumpen eingesetzt, um die Spülflüssigkeit wieder abzupumpen. Im typischen Ablauf solcher minimal-invasiver Eingriffe ist ein Einsatz weiterer medizintechnischer Vorrichtungen zum Schneiden, Abtragen oder Veröden vorgesehen, die jeweils über eine eigene Absaugleitung verfügen. Sind alle diese Absaugleitungen unverschlossen, kann kein Druck aufgebaut werden um die Körperhöhle aufzudehnen. Typischerweise sollen möglichst wenige Einschnitte in die Wände der Körperhöhle erfolgen und daraus resultiert, dass eine Zuführleitung mit mehreren Absaugleitungen kombiniert ist.

Bei therapeutischen Eingriffen in Körperhöhlen (z.B. Knie- oder Schultergelenk, Blase, Gebärmutter oder andere künstliche Körperhöhlen) werden häufig zwei medizinische Instrumente verwendet, nämlich ein Shaver und eine Hochfrequenz (HF)-Ablationseinrichtung. Weiterhin hat es sich als vorteilhaft erwiesen, wenn beide medizinische Instrumente mit einer Absaugleitung verbunden sind. Als Herausforderung hat sich dabei die Steuerung der Saugleistung erwiesen. Einerseits muss das abgesaugte Flüssigkeitsvolumen dem zuströmenden Volumen entsprechen, da sonst entweder die nötige Aufdehnung der Körperhöhle nicht mehr gewährleistet ist oder andernfalls der Druck in der Körperhöhle so stark steigt, dass es zu Gewebeverletzungen kommen kann. Andererseits ist es sinnvoll, dass das abgepumpte Volumen insbesondere über die Abführleitung des jeweiligen Instrumentes erfolgt.

Weiterhin ist es Stand der Technik, die Absaugleitungen durch sogenannte Quetschventile (pinch valves) mit einer Magnetspule bzw. einem Linearmagneten (Solenoid) durch einen Stempel zu quetschen und somit zu verschließen. Dies erfüllt die Randbedingung, dass das zur Aufdehnung (Distension) genutzte Medium nicht mit dem Gerät in Berührung kommt und das Medium somit über einen sterilen Schlauch bis zur Körperhöhle gelangt. Die Kraft mit der die Magnetspule auf den Schlauch einwirkt, ist jedoch direkt proportional mit der Geschwindigkeit, d.h. bei hoher Schaltgeschwindigkeit ergibt sich eine hohe Belastung für das Widerlager des Schlauches und das Schlauchmaterial.

EP 0173816 A2 beschreibt eine Spülvorrichtung für die endoskopische Untersuchung von Harnleitern mit zwei parallel angeordneten Kolbenpumpen, deren Kolbenstangen mit Zahnstangen verbunden sind.

US 2012/0090620 A1 beschreibt eine Vorrichtung zum Spülen von Körperhöhlen mit zwei Pumpen, die über ein manuelles Bedienteil gesteuert werden, so dass die zweite Pumpe Flüssigkeit aus der Vorrichtung in die Körperhöhle pumpt, während die erste Pumpe Flüssigkeit aus der Körperhöhle in die Vorrichtung ansaugt.

EP 1 374 925 B1 beschreibt eine Vorrichtung zum Spülen eines Körperhohlraumes mit einer Spülpumpe und einer Saugpumpe, wobei die Spülpumpe in Abhängigkeit der Soll-Flussrate im Körperhohlraum und die Saugpumpe in Abhängigkeit des Soll-Druckes im Körperhohlraum gesteuert wird und wobei die Drehzahl der Saugpumpe als Gebersignal für die Steuerung der Spülpumpe dient.

Die nachfolgend beschriebene medizinische Vorrichtung gewährleistet auf einfache Art, dass die oben genannten Aufgaben gelöst werden: Es wird exakt so viel Volumen aus der Körperhöhle abgepumpt, wie zugeführt wird. Weiterhin erfolgt das Abpumpen hauptsächlich über die Abführleitung des jeweils betriebenen Instrumentes. Ferner wurde eine verbesserte Quetschventilsteuerung entwickelt.

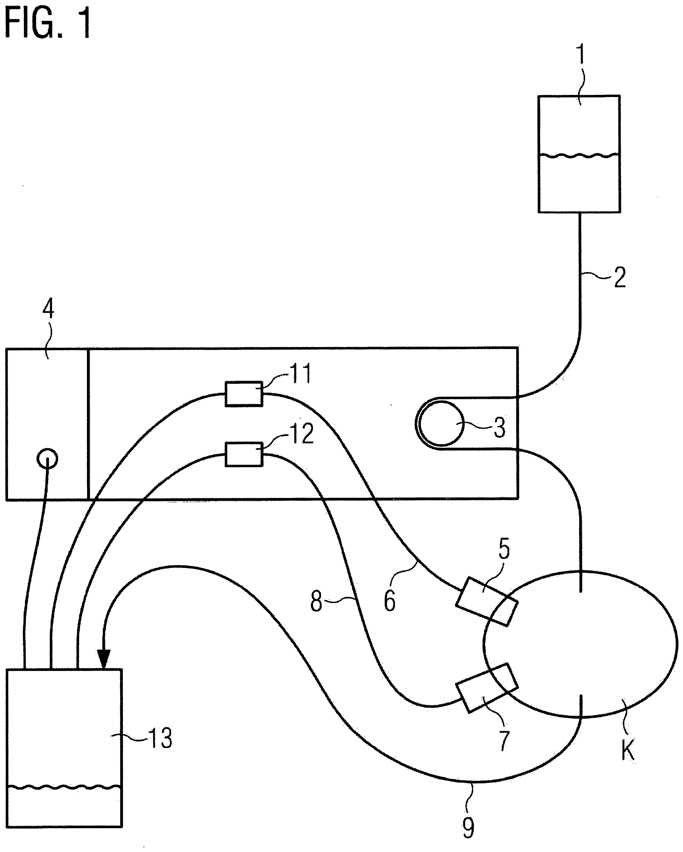

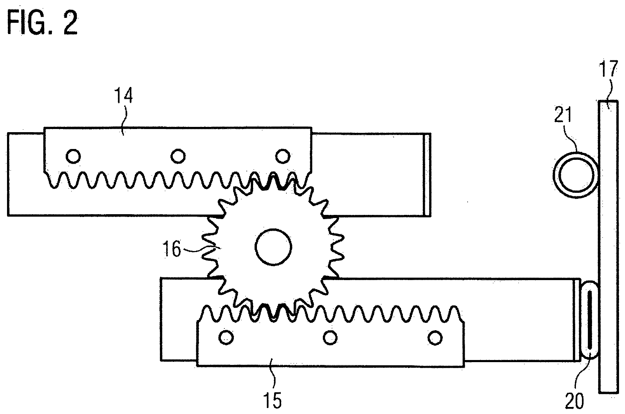

Die erfindungsgemäße Vorrichtung ist in den Figuren 1 und 2 beschrieben, auf die hiermit Bezug genommen wird.

Im Rahmen der erfindungsgemäßen Vorrichtung ist zunächst ein Vorratsbehälter für die Spülflüssigkeit eingerichtet. Die Zuführleitung aus diesem Vorratsbehälter führt über eine gesteuerte Rollenradpumpe in die Körperhöhle. Die Ankoppelung der Zuführleitung an das Rollenrad erfolgt zweckmäßigerweise durch eine entsprechende Kassette, wie sie im Stand der Technik bekannt ist. Zur Absaugung aus der Körperhöhle dient eine gesteuerte Vakuumpumpe. Die beiden medizinischen Instrumente (z.B. Shaver und Hochfrequenz-Ablationseinrichtung) weisen jeweils eine Absaugleitung auf. Die beiden Leitungen sind jeweils mit einem Quetschventil versehen, welches weiter unten näher beschrieben wird. Die Abführleitungen führen in Strömungsrichtung nach den Quetschventilen in einen Vorratsbehälter. Dieser Vorratsbehälter ist mit der gesteuerten Vakuumpumpe verbunden. Der Vorratsbehälter ist so eingerichtet, dass sich die abgesaugte Flüssigkeit in ihm sammelt und nicht in die Vakuumpumpe gelangt.

Im Regelfall ist noch eine weitere Leitung zwischen der Körperhöhle und dem Vorratsbehälter eingerichtet. Diese zusätzliche Leitung enthält eine Drossel, so dass in jedem Fall nur sehr wenig Spülflüssigkeit abgesaugt wird (weniger als 10% des Gesamtvolumenstroms). Die Drosselung des Volumenstroms kann auch durch einen entsprechend verringerten Schlauchdurchmesser erfolgen. Die zusätzliche Leitung dient lediglich der Erzeugung eines konstanten Durchflusses, um anfallende kleine Blutungen und Gewebetrümmer abzuführen und so eine gute Sicht in der fluidgefüllten Körperhöhle zu ermöglichen.

Ein wichtiges Element der beschriebenen medizintechnischen Vorrichtung sind die Quetschventile. Diese sind so miteinander gekoppelt, dass eine Leitung vollständig geöffnet ist, wenn die andere Leitung vollständig geschlossen ist. Hierzu dient ein Schrittmotor, der über ein Zahnrad zwei Zahnstangen antreibt. Die beiden Zahnstangen sind jeweils gegenüberliegend an dem Zahnrad positioniert, so dass eine Zahnstange aus dem Gerät ausfährt, wenn die andere eingefahren wird (antiparallele Betriebsweise). Die beiden Schläuche sind jeweils in einer Klemmvorrichtung positioniert, die gleichzeitig als Widerlager für die als Quetschvorrichtung dienenden Zahnstangen dient. Durch Ansteuerung des Schrittmotors kann ausgewählt werden, durch welche Leitung die Spülflüssigkeit abgesaugt wird. In den jeweiligen Endlagen erfolgt die Absaugung entweder vollständig über die eine Leitung oder vollständig über die andere Leitung (Volumenstromverhältnis 100:0 oder 0:100). Es sind jedoch auch Zwischenstellungen einstellbar, so dass beispielsweise der Flüssigkeitsstrom im Verhältnis 80:20 oder 60:40 aufgeteilt wird.

Die fest mit den Zahnstangen verbunden Quetschelemente wirken auf die abzuklemmenden Schlauchabschnitte ein und üben analog zum Stand der Technik einen Druck aus, der zu einer Verformung bis hin zu einem Verschluss des Schlauchlumens führt.

Ein optionales Erfindungsmerkmal ist ein am Zahnrad befindlicher Positionssensor, der zu jedem Zeitpunkt eine absolute Positionsbestimmung des Zahnrades ermöglicht. Vorteilhaft ist hierbei, dass auch im Stillstand die Position ermittelt werden kann und keine Bewegung am Positionssensor erforderlich wird, die ein teilweises oder kurzfristiges Öffnen des abgeklemmten Schlauchlumens zur Folge haben würde. Diese Eigenschaft wird genutzt für ein weiteres Erfindungsmerkmal, eine im Ablauf der Verschlussphase variablen Antriebssteuerung für das Zahnrad, dergestalt, dass die Zahnstangen in der Umschaltphase maximal schnell bewegt werden und in der Abklemmphase bzw. einem Teil der Abklemmphase bis zum endgültigen Verschluss die Bewegung mit einer Antriebsleistung erfolgt, die einem definierten und vorbestimmten Druck auf den abzuklemmenden Schlauch entspricht. Um dies zu erreichen, wird, ebenfalls erfindungsgemäß, die Antriebsvorrichtung dergestalt ausgelegt, dass eine dort nutzbare Motor-Getriebe-Kombination dergestalt ausgelegt wird, dass bei der minimal benötigten Kraft zum Abklemmen die maximal mögliche Geschwindigkeit erreichbar ist in den Umschaltphasen.

Erfindungsgemäß wählt man eine Motor-Getriebe-Kombination, die eine maximal erreichbare Quetschkraft erreichen kann. Durch die Kopplung von steigender Kraftübertragung und sinkender Verfahrgeschwindigkeit in Getrieben wird eine bei voller Drehzahl des Motors erreichbare Verfahrgeschwindigkeit durch die Definition der maximal erreichbaren Quetschkraft festgelegt. Vorausgesetzt wird, dass die eingesetzten Bauteile nicht überdimensioniert sind und damit so preiswert wie möglich sind. Die erfindungsgemäße Auswahlrichtlinie ist daher, zuerst die Motor-Getriebe-Kombination zur Bewegung der Zahnstangen so zu dimensionieren, dass gerade eben die benötigte Druckkraft erzeugt werden kann, um die Schläuche abzuklemmen und somit sicher zu verschließen. Damit wird die maximal erreichte Verfahrgeschwindigkeit der Motor-Getriebe-Kombination höher sein, als bei Motor-Getriebe-Kombinationen, die eine stärkere Druckkraft erzeugen. Die benötigte Druckkraft kann über eine Messung des Druckes auf dem abzuklemmenden Schlauch bis zu dessen Verschluss bestimmt werden.

Optional kann der Positionssensor auch an einer der Zahnstangen befestigt sein. Möglich ist auch der Einsatz mehrerer Positionssensoren (z.B. an Zahnstange und Zahnrad oder an beiden Zahnstangen).

Ein Vorteil der erfindungsgemäßen Ventilsteuerung ist, dass eine Gestaltung der Schlauchabschnitte und der Widerlager derart erfolgen kann, dass weniger robustes und damit preiswerteres Material verwendet oder Material eingespart werden kann und gleichzeitig ein maximal schneller Verschluss der Saugleitungen erfolgen kann.

Die erfindungsgemäße Ventilsteuerung erfolgt derart, dass die über den Positionssensor ermittelte aktuelle Position der Zahnstangen die Verfahrgeschwindigkeit beeinflusst. Die Verfahrgeschwindigkeit ist in den Phasen maximal, in denen kein Kontakt der Quetschelemente bzw. Zahnstangen mit dem abzuquetschenden Schlauch besteht. Auf diese maximale Verfahrgeschwindigkeit wird die Antriebseinheit ausgelegt. Gemäß eines erfindungsgemäßen Geschwindigkeitsprofils wird eine Reduzierung der Geschwindigkeit auf 50% in den letzten 10 bis 20% des Weges, d.h. bis der Schlauch komplett abgequetscht wird, vorgenommen. Mit dieser Reduktion der Zudrückgeschwindigkeit des Schlauchlumens werden Druckstöße in der Saugleitung vermieden und das Schlauchmaterial und Widerlager geschont, womit sich die Lebensdauer von Schlauch und der den Schlauch führenden Kassette bzw. Schlauch-Einlegehilfe verlängert. Dies ist mit Linearmagneten nicht möglich. Andere Werte für die Verfahrgeschwindigkeitsreduktion und die Zeitpunkte wo die Verfahrgeschwindigkeit geändert wird, inklusive stufenlosen Änderungen und mehreren Stufen sind ebenfalls erfindungsgemäß.

Eine Weiterführung des Erfindungsgedankens ist ein Verfahrgeschwindigkeitsprofil, in dem für einen kurzen Weg vor dem Schlauchkontakt die Verfahrgeschwindigkeit reduziert wird, bei Schlauchkontakt eine Erhöhung der Verfahrgeschwindigkeit erfolgt mit nachfolgender stufenloser Reduktion auf Null in der Position, wo der Schlauch komplett abgeklemmt ist. Andere Profile mit beliebigen Kombinationen von Erhöhung und Reduktion der Verfahrgeschwindigkeit sind ebenfalls erfindungsgemäß.

Zu Beginn der Behandlung wird zunächst über die Rollenradpumpe Spülflüssigkeit in die Körperhöhle gepumpt, bis der nötige Druck bzw. die nötige Aufdehnung der Körperhöhle erreicht ist. Wenn der nötige Druck erreicht ist, wird die Saugpumpe aktiviert, wobei die Leistung von Rollenradpumpe und Saugpumpe gekoppelt sind. Es wird jeweils nur so viel Flüssigkeit abgepumpt, wie zugeführt wird. Bei Aktivierung der medizinischen Instrumente (z.B. Shaver und Hochfrequenz-Ablationsvorrichtung) werden die Quetschventile jeweils so eingestellt, dass die Pumpleistung hauptsächlich über die Abflussleitung des jeweiligen Instrumentes führt. Wenn der Shaver aktiviert ist, wird die an den Shaver angeschlossene Abflussleitung geöffnet und die an die Hochfrequenz-Ablationsvorrichtung angeschlossene Leitung geschlossen. Wenn zu einem späteren Zeitpunkt die andere medizinische Vorrichtung benutzt wird, wird die andere Leitung geöffnet und die erste Leitung geschlossen. Wie oben bereits beschrieben, kann das Öffnen bzw. Schließen auch teilweise erfolgen. In diesem Fall würden beispielsweise 80% der Saugleistung durch die an das aktivierte medizinische Instrument angeschlossene Saugleitung erfolgen, während 20% der Saugleistung über das andere Instrument geführt würde. Am Schluss der Prozedur besteht die Möglichkeit, die Rollenradpumpe zu stoppen, aber die Saugpumpe so lange zu aktivieren, bis die Spülflüssigkeit so weit wie möglich abgesaugt wurde.

### Bezugszeichenliste:

- (1): Vorratsbehälter für die Spülflüssigkeit,
- (2): Zuführleitung
- (3): gesteuerte Rollenradpumpe
- (4): gesteuerte Vakuumpumpe
- (5): erstes medizinisches Instrument
- (6): ersten Absaugleitung
- (7): zweites medizinisches Instrument
- (8): zweiten Absaugleitung
- (9): dritte Absaugleitung (optional)
- (10): Drossel an optionaler dritter Absaugleitung
- (11): erstes Quetschventil
- (12): zweites Quetschventil
- (13): Abfallbehälter
- (14): erste Zahnstange mit erstem Quetschelement
- (15): zweite Zahnstange mit zweitem Quetschelement
- (16): Zahnrad
- (17): Widerlager für Quetschelement an erster Zahnstange mit eingelegtem und abgeklemmten Schlauch (20)
- (18): Widerlager für Quetschelement an zweiter Zahnstange mit eingelegtem nicht abgeklemmten Schlauch (21)
- (19): Positionssensor

## Patentansprüche

1. Vorrichtung zum Durchspülen einer Körperhöhle (K), enthaltend
(i) Vorratsbehälter (1) für die Spülflüssigkeit,
(ii) Zuführleitung (2)
(iii) gesteuerte Rollenradpumpe (3)
(iv) gesteuerte Vakuumpumpe (4)
(v) erstes medizinisches Instrument (5) mit einer ersten Absaugleitung (6)
(vi) zweites medizinisches Instrument (7) mit einer zweiten Absaugleitung (8)
(vii) optional eine dritte Absaugleitung (9) mit einer Drossel (10),
(viii) erstes Quetschventil (11) an der ersten Absaugleitung (6)
(ix) zweites Quetschventil (12) an der zweiten Absaugleitung (8) versehen,
(x) Abfallbehälter (13) , verbunden mit erster Absaugleitung (6), zweiter Absaugleitung (8) und optionaler dritter Absaugleitung (9) sowie mit der gesteuerten Vakuumpumpe (4),
**dadurch gekennzeichnet, dass** das erste Quetschventil (11) und das zweite Quetschventil (12) antiparallel miteinander gekoppelt ist.

2. Vorrichtung gemäß Anspruch 1, **dadurch gekennzeichnet, dass** die antiparallele Koppelung so erfolgt, dass die beiden Quetschventile (11, 12) durch Quetschelemente gebildet werden, die jeweils auf Zahnstangen (14, 15) befestigt sind und die beiden Zahnstangen mittels eines Zahnrades (16) miteinander gekoppelt sind, so dass sie eine antiparallele Bewegung ausführen.

3. Vorrichtung gemäß Anspruch 2, wobei das Zahnrad (16) durch einen Schrittmotor angetrieben wird.

4. Vorrichtung gemäß Anspruch 2 oder 3, wobei das Zahnrad (16) einen Positionssensor enthält, der zu jedem Zeitpunkt eine absolute Positionsbestimmung des Zahnrades ermöglicht.

5. Vorrichtung gemäß einem der Ansprüche 2 bis 4, wobei ein oder beide Zahnstangen ein oder mehrerer Positionssensoren enthält, die zu jedem Zeitpunkt absolute Positionsbestimmungen der Zahnstangen ermöglichen.

6. Vorrichtung gemäß einem der Ansprüche 1 bis 5, wobei die dritte Absaugleitung (9) eine Drossel enthält, so dass durch die die dritte Absaugleitung (9) weniger als 10% des Gesamtvolumenstroms abgesaugt wird.

7. Vorrichtung gemäß einem der Ansprüche 1 bis 6, wobei das erste und das zweite medizinische Instrument (5, 7) durch einen Shaver und eine Hochfrequenz-Ablationseinrichtung gebildet werden, die jeweils mit einer Absaugleitung (6, 8) verbunden sind.

## Claims

1. A device for irrigating a body cavity (K), including
(i) a reservoir (1) for the irrigation fluid,
(ii) a feed line (2)
(iii) a controlled roller pump (3)
(iv) a controlled vacuum pump (4)
(v) a first medical instrument (5) with a first suction line (6)
(vi) a second medical instrument (7) with a second suction line (8)
(vii) optionally a third suction line (9) with a restrictor (10)
(viii) a first pinch valve (11) at the first suction line (6)
(ix) a second pinch valve (12) provided at the second suction line (8)
(x) a waste container (13) connected to the first suction line (6), the second suction line (8) and optionally the third suction line (9) as well as to the controlled vacuum pump (4),
**characterized in that** the first pinch valve (11) and the second pinch valve (12) are coupled with each other in an anti-parallel manner.

2. The device according to Claim 1, **characterized in that** the anti-parallel coupling is carried out such that the two pinch valves (11, 12) are formed through pinch elements which are each attached to gear racks (14, 15) and the two gear racks are coupled with each other via a gear wheel (16) so that they perform an anti-parallel motion.

3. The device according to Claim 2, wherein the gear wheel (16) is driven by a stepper motor.

4. The device according to Claim 2 or 3, wherein the gear wheel (16) includes a position sensor which allows for an absolute position determination of the gear wheel at all times.

5. The device according to any one of Claims 2 to 4, wherein one or both gear rack(s) include(s) one or more position sensors which allow for absolute position determination of the gear wheels at all times.

6. The device according to any one of Claims 1 to 5, wherein the third suction line (9) includes a restrictor so that less than 10% of the total volume flow is suctioned through the third suction line (9).

7. The device according to any one of Claims 1 to 6, wherein the first and third medical instruments (5, 7) are formed by a shaver and a high-frequency ablation device, each of which is respectively connected to a suction line (6, 8).

## Revendications

1. Dispositif destiné à rincer une cavité corporelle (K), contenant
(i) un réservoir (1) pour le liquide de rinçage,
(ii) une conduite d'alimentation (2)
(iii) une pompe à roue à roulement commandée (3)
(iv) une pompe à vide commandée (4)
(v) un premier instrument médical (5) avec une première conduite d'aspiration (6)
(vi) un second instrument médical (7) avec une deuxième conduite d'aspiration (8)
(vii) en option une troisième conduite d'aspiration (9) avec un étranglement (10),
(viii) une première vanne à pincement (11) sur la première conduite d'aspiration (6)
(ix) une seconde vanne à pincement (12) prévue sur la deuxième conduite d'aspiration (8),
(x) un contenant à déchets (13), relié à la première conduite d'aspiration (6), à la deuxième conduite d'aspiration (8) et en option à la troisième conduite d'aspiration (9) ainsi qu'à la pompe à vide commandée (4),
**caractérisé en ce que** la première vanne à pincement (11) et la seconde vanne à pincement (12) sont couplées l'une à l'autre de manière antiparallèle.

2. Dispositif selon la revendication 1, **caractérisé en ce que** le couplage antiparallèle s'effectue de telle sorte que les deux vannes à pincement (11, 12) soient formées par des éléments de pincement qui sont fixés respectivement sur des crémaillères (14, 15), et les deux crémaillères sont couplées l'une à l'autre à l'aide d'un pignon (16) afin qu'elles réalisent un mouvement antiparallèle.

3. Dispositif selon la revendication 2, dans lequel le pignon (16) est entraîné par un moteur pas à pas.

4. Dispositif selon la revendication 2 ou 3, dans lequel le pignon (16) contient un capteur de position qui permet de déterminer la position absolue du pignon à chaque instant.

5. Dispositif selon une des revendications 2 à 4, dans lequel un ou les deux pignon(s) contient/contiennent un ou plusieurs capteur(s) de position qui permet(tent) de déterminer les positions absolues des crémaillères à chaque instant.

6. Dispositif selon une des revendications 1 à 5, dans lequel la troisième conduite d'aspiration (9) contient un étranglement afin que moins de 10 % du débit volumique total ne soit aspiré par la troisième conduite d'aspiration (9).

7. Dispositif selon une des revendications 1 à 6, dans lequel le premier et le second instrument médical (5, 7) sont formés d'un rasoir et d'un moyen d'ablation à haute fréquence, lesquels sont reliés respectivement à une conduite d'aspiration (6, 8).
